# FASCICULE DE BREVET EUROPEEN

(11) **EP 0 814 835 B1**
(45) Date de publication et mention de la délivrance du brevet: **05.11.2003**
(21) Numéro de dépôt: 95929918.1
(22) Date de dépôt: 31.08.1995
(51) Int. Cl.: A61K 39/39, A61K 39/245, C07K 19/00, C07K 14/045, C12N 15/38, C12N 15/62

(54) **PROCEDE POUR AUGMENTER L'IMMUNOGENICITE, PRODUIT OBTENU ET COMPOSITION PHARMACEUTIQUE**
VERFAHREN ZUR STEIGERUNG DER IMMUNOGENIZITÄT, ERHALTENES PRODUKT UND ARZNEIMITTEL
METHOD FOR ENHANCING IMMUNOGENICITY, PRODUCT OBTAINED AND PHARMACEUTICAL COMPOSITION

(30) Priorité: 31.08.1994 FR 9410479
(43) Date de publication de la demande: 07.01.1998
(73) Titulaire: Biovector Therapeutics S.A., 31676 Labege Cedex (FR); INSTITUT NATIONAL DE LA SANTE ET DE LA RECHERCHE MEDICALE (INSERM) (E.P.S.T.), 75013 Paris (FR)
(72) Inventeur: BETBEDER, Didier, F-31140 Aucamville (FR); DAVRINCHE, Christian, F-31700 Cornebarrieu (FR); DAVIGNON, Jean-Luc, F-31170 Tournefeuille (FR); PRIEUR, Eric Bâtiment A1, Appartement 13, F-31400 Toulouse (FR)
(74) Mandataire: Pernez, Helga
(86) Numéro de dépôt international: FR9501138
(87) Numéro de publication internationale: WO96006638

(56) Documents cités:
- EP-A- 0 344 040
- WO-A-94/20078
- N.van ROOIJEN "LIPOSOMES" IN I.M. ROITT et al.(Ed's) "ENCYCLOPEDIA OF IMMUNOLOGY", ACADEMIC PRESS, LONDON, 1992
- BIOCHEMICAL AND BIOPHYSICAL RESEARCH COMMUNICATIONS, vol. 195, no. 1, 31 Août 1993 ORLANDO, FL US, pages 469-477, C. DAVRINCHE ET AL. 'EXPRESSION OF HUMAN CYTOMEGALOVIRUS IMMEDIATE EARLY PROTEIN IE1 IN INSECT CELLS: SPLICING OF RNA AND RECOGNITION BY CD4+ T-CELL CLONES.' cité dans la demande
- GENE, vol. 67, 1988 AMSTERDAM NL, pages 31-40, D.B. SMITH ET AL. 'SINGLE-STEP PURIFICATION OF POLYPEPTIDES EXPRESSED IN ESCHERICHIA COLI AS FUSIONS WITH GLUTATHIONE S-TRANSFERASE.' cité dans la demande

## Description

La présente invention se rapporte à un procédé permettant d'augmenter l'immunogénicité d'un antigène et à l'utilisation des produits susceptibles d'être obtenus.

Le cytomégalovirus humain (CMV), un herpès virus à l'état latent chez les individus immunocompétents est responsable de pathologies très diverses et souvent mortelles en cas d'immunodépression (foetus, greffe, Sida, leucémie, cancer) (pour revue voir (1)). Le CMV est la source principale de malformations congénitales d'origine infectieuse. Pour toutes ces raisons le CMV pose un grave problème de santé publique. La lutte contre ce virus est limitée à la chimiothérapie par des antiviraux non spécifiques comme Ganciclovir ® et Foscarnet ®, dont les effets secondaires et la néphrotoxicité ont été décrits. En matière de prévention, l'immunothérapie passive (injection de gammaglobulines) diminue l'incidence de la primo-infection chez les greffés de moelle séronégatifs. Chez les transplantés (allogreffes de moelle, de rein, de coeur, de foie), l'infection à CMV est associée au rejet du greffon. Les receveurs, sous immunosuppresseur (cyclosporine) sont traités au Ganciclovir ® pendant la greffe et reçoivent des gammaglobulines pendant les trois mois suivant l'intervention. L'injection intra veineuse à haute dose d'immunoglobulines chez ces patients a permis, dans certains cas, une réduction de la fréquence des pneumonies et des rejets. Ceci soulève le problème non négligeable du coût très élevé associé à ces traitements. L'infection opportuniste par CMV chez les individus HIV+ est une des plus dramatiques et nécessite une chimiothérapie par des antiviraux.

Les économies réalisées en élaborant une politique de prévention des maladies liées à l'infection par CMV chez les individus à risque seraient substantielles. En effet, des estimations du coût associé à la vaccination d'un individu et à sa prise en charge (analyses sérologiques, vaccin, traitement d'effets secondaires mineurs) effectuées aux Etats Unis (2) montrent que ce coût serait environ 50 fois inférieur à celui des soins apportés à un nouveau-né victime d'une infection congénitale. L'infection à CMV est observée chez les 2/3 des transplantés rénaux et à une fréquence élevée chez les autres transplantés. Si elle est associée à des complications chez environ 1/3 d'entre eux, le coût annuel ajouté à celui de la transplantation est considérable. Malgré l'impact de drogues comme le Ganciclovir ® ou l'injection de gammaglobulines sur la maladie, la prévention de l'infection primaire et de la réactivation chez ces patients doit être une priorité. Les bénéfices de l'immunisation sont évidents tant sur le plan clinique qu'économique.

La mise au point d'un vaccin anti-CMV devrait permettre d'enrayer le développement des pathologies associées aux infections congénitales en vaccinant les jeunes femmes avant et pendant la grossesse, d'assurer la protection des malades en attente de greffes et d'initier une réponse anti-cytomégalovirus chez les individus HIV+ asymptomatiques. L'utilisation de virus tué ne semble pas appropriée car les extraits viraux n'induisent pas, en général, de réponse de type TCD8+ cytotoxique. Le virus atténué soulève le problème du caractère oncogène, de la latence et de la réactivation des particules virales. Le développement de vaccins recombinants qui devrait permettre d'éviter ces risques, nécessite la connaissance des antigènes ou fragments d'antigènes (épitopes) les plus immunogènes de l'agent infectieux. Le but de la vaccination est l'induction d'une immunité protectrice. Une approche rationnelle de la vaccination doit impliquer trois étapes : (i) l'identification des mécanismes effecteurs responsables de la protection, (ii) le choix d'un antigène capable d'induire une réponse chez tous les individus, et (iii) l'utilisation d'une voie d'administration du vaccin capable d'induire le type de réponse souhaité (humorale: anticorps, cellulaire: cytotoxique et auxiliaire).

La défense de l'organisme contre une infection virale est assurée essentiellement par le développement d'une réponse immunitaire humorale (production d'anticorps neutralisants) empêchant l'adsorption du virus à la surface cellulaire d'une part et d'une réponse cellulaire d'autre part (cellules TCD8+ cytotoxiques et TCD4+ auxiliaires) éliminant les cellules infectées et inhibant la réplication virale (synthèse de cytokines (TNFα, IFNγ...)). En ce qui concerne le cytomégalovirus humain, parmi les 200 protéines codées par l'ADN double brin (230 kpb), trois d'entre elles sont les cibles majeures respectives de ces différents types de réponse: la glycoprotéine d'enveloppe gB (3), la phosphoprotéine du tégument pp65 (4) et la phosphoprotéine de régulation IE1 (5).

Si la mise au point d'un vaccin anti-CMV semble être une nécessité, il subsiste le problème général de la vectorisation des antigènes recombinants. Les antigènes introduits dans des structures synthétiques doivent être capables d'initier ou de restaurer une réponse immunitaire B et T (auxiliaire CD4+ et cytotoaique CD8+) durable, assurant ainsi la protection des individus contre une infection primaire, une réinfection ou une réactivation du virus latent. L'activation des cellules T (cellules naives ou cellules mémoires) est liée à la capacité de présentation de l'antigène par les cellules présentatrices d'antigène dont les plus importantes sont les cellules dendritiques, les lymphocytes B et les macrophages. Dans ce contexte les particules vectrices devront permettre l'accès aux voies de présentation endogène (classe 1, TCD8+) et exogène (classe II, TCD4+).

La société Biovector Therapeutics a développé un type de structure appelée Biovecteur Supramoléculaire constitué d'un noyau polysaccharidique (NPS) recouvert d'une ceinture d'acides gras (CA) ou de phospholipides (BVSM.) dont on peut faire varier la composition. La maille polysaccharidique possède un degré de réticulation modulable, peut être fonctionnalisée (radical anionique ou cationique), est très stable, et permet la fixation d'une quantité importante d'antigène à l'intérieur du noyau aussi bien qu'à sa périphérie.

De manière inattendue, la Demanderesse a trouvé que l'association d'une protéine ou d'un peptide avec ces vecteurs permettait d'observer une potentialisation de la réponse immunogène par rapport à celle entraînée par l'administration de l'antigène seul.

C'est pourquoi la présente invention a pour objet un procédé pour augmenter l'immunogénicité d'un antigène, caractérisé en ce que l'antigène est associé par des liaisons ioniques et/ou hydrophobes à un vecteur particulaire, ledit vecteur comportant :
- un noyau hydrophile non liquide
- une couche externe constituée de composés lipidiques choisis dans le groupe comprenant les phospholipides et les acides gras.

De préférence, le noyau est constitué d'une matrice de polysaccharides ou d'oligosaccharides naturellement ou chimiquement réticulés. Selon l'un des aspects de l'invention, des ligands ioniques sont greffés sur le noyau, ce ligand pouvant notamment porter une fonction choisie dans le groupe comprenant : phosphates, sulfates, acides carboxyliques, ammonium quaternaires, amines secondaires, amines primaires.

Les vecteurs permettant la mise en oeuvre du procédé peuvent avoir une taille comprise entre 10 nm et 5 µm, et des résultats particulièrement avantageux sont obtenus pour des tailles comprises entre environ 25 nm et 200 nm, notamment de l'ordre de 80 nm.

L'association d'un antigène à des vecteurs particulaires acylés ou phospholipidés augmente notamment la réponse proliférative de clones de cellules TCD4+ *in vitro* à cet antigène, en présence de leucocytes du sang périphérique et de lymphocytes B/EBV isolés. Les rendements d'association de l'antigène aux particules sont élevés et les complexes obtenus sont très stables en milieu physiologique. La mise en évidence d'une potentialisation de la réponse proliférative T souligne les propriétés remarquables de ces biovecteurs et l'intéret de leur utilisation potentielle en vaccination.

Ces résultats ne sont pas obtenus avec un simple mélange, sans interaction préalable, de l'antigène et du vecteur particulaire.

L'antigène est associé au vecteur particulaire par des liaisons ioniques et/ou hydrophobes.

La couche externe du vecteur particulaire est constituée d'acides gras naturels fixés au noyau par des liaisons covalentes. Selon un autre aspect, cette couche externe est constituée de phospholipides naturels ou synthétiques.

L'invention a également pour objet un produit susceptible d'être obtenu par le procédé décrit, et qui comprend une association potentialisant l'immunogénicité, d'un antigène lié par des liaisons ioniques et/ou hydrophobes à un vecteur particulaire, ledit vecteur particulaire comportant
- un noyau hydrophile non liquide et,
- une couche externe, associée au noyau par des interactions hydrophobes et/ou des liaisons ioniques, et constituée des composés choisis dans le groupe comprenant les phospholipides et les acides gras. Dans ce produit potentialisant l'immunogénicité l'antigène est une protéine du CMV.

Les propriétés remarquables de ces vecteurs peuvent en effet être exploitées pour l'encapsulation d'antigènes du CMV humain, dans le but de concevoir un vaccin recombinant qui associerait les antigènes IE1, pp65 et gB pour les raisons évoquées plus haut. Un tel produit serait capable d'engendrer une réponse humorale et cytotoxique.

L'antigène peut donc notamment être la protéine IE1 du CMV, ou un fragment de cette protéine.

Un fragment de la protéine virale recombinante IE1 du Cytomégalovirus humain (souche Towne) a été produit et purifié, sous la forme d'une protéine de fusion (GST-e4) dans E. Coli. L'association de la protéine à différents types de particules (NPS, CA et BVSM) de 80nm est très stable et les rendements très élevés. Nous avons décrit un effet adjuvant des complexes antigène (Ag)/ particules sur la réponse proliférative de clones TCD4+ spécifiques *in vitro.* Cet effet a été observé en utilisant comme cellules présentatrices d'antigène des leucocytes du sang périphérique et des lymphocytes B transformés par EBV (B/EBV).

La prolifération de clones T anti IE1 en présence de GST-e4 recombinante soluble ou associée à des particules suggère que le couplage de l'antigène viral à la GST bactérienne, l'absence de phosphorylation de la protéine dans ce système d'expression procaryote, et l'association à des biovecteurs n'ont pas eu d'influence sur la nature des peptides épitopes générés par les cellules présentatrices. L'une des hypothèses, par laquelle la Demanderesse n'entend aucunement limiter l'invention, est que l'effet adjuvant observé lorsque l'antigène est associé aux particules pourrait être corrélé à une capture plus importante de protéine, par la cellule présentatrice que lorsque l'antigène est soluble, comme cela a été constaté par la Demanderesse. La concentration de l'antigène dans les compartiments endo-lysosomiaux pourrait favoriser l'interaction des molécules de classe II avec des peptides viraux plutôt qu'avec des peptides endogènes ou exogènes issus de protéines du sérum par exemple. Ceci aurait pour conséquence l'expression d'un plus grand nombre de complexes DR-peptide IE1 à la surface cellulaire. L'effet potentialisateur observé avec les CA pourrait être lié à une meilleure accessibilité de l'antigène. Les vecteurs que nous avons utilisés ont permis l'incorporation d'une masse importante d'antigène par simple mélange en solution aqueuse. Ceci représente un avantage important par rapport aux protocoles d'encapsulation dans d'autres types de particules comme les liposomes (6).
Il est possible que l'effet adjuvant observé puisse aussi être médié par une opsonisation des particules, faisant intervenir l'interaction entre des IgG sériques ou des fragments du complément comme C3b et leurs récepteurs respectifs à la surface des cellules présentatrices.

La Demanderesse a montré qu'une protéine de fusion plus stable que e4 seul, entre le fragment e4 du CMV et la glutathion-S-transférase (GST), préparée par génie génétique peut être utilisée dans les vecteurs selon l'invention, sans nécessiter une étape de clivage afin d'éliminer la partie GST. Ceci représente un progrès considérable car une telle protéine est aisément purifiable et la suppression de l'étape de clivage permet d'augmenter le rendement et d'envisager sa production à grande échelle.

Le but de la vaccination est d'induire la maturation de cellules impliquées dans l'élimination spécifique d'un agent pathogène et de maintenir des clones de cellules mémoires spécifiques des antigènes cibles dans la circulation et dans les organes lymphoides secondaires. La contribution importante des cellules B en tant que cellules présentatrices d'antigène à l'activation des cellules T a été rapportée (7). Il a été montré que la cellule B n'endocyte efficacement les antigènes solubles, à faible concentration, que par l'intermédiaire de son immunoglobuline de surface. Les travaux destinés à cibler des liposomes sur des lymphocytes B faisaient appel à la fixation à leur surface d'anticorps monoclonaux (8) destinés à interagir avec les immunoglobulines de surface de ces cellules. La fréquence des lymphocytes B spécifiques étant très faible, l'effet observé ici en utilisant des particules devrait permettre d'amplifier la réponse T spécifique à l'antigène complexé sans passer par une reconnaissance par les lymphocytes B spécifiques. La présentation d'un Ag aux cellules TCD4+ activée induit, outre leur prolifération, la sécrétion de cytokines et l'expression de molécules de surface importantes pour la prolifération et la différentiation des cellules B en plasmocytes sécrétant des anticorps.

Selon un autre aspect, l'invention a pour objet une utilisation d'un vecteur particulaire pour la préparation d'un médicament utile pour augmenter l'immunogénicité d'un antigène. Cette utilisation comprend un antigène associé à un vecteur particulaire selon les modalités définies précédemment.

Les exemples qui suivent sont destinés à illustrer l'invention sans aucunement en limiter la portée. Dans ces exemples, on se réfèrera aux figures suivantes :

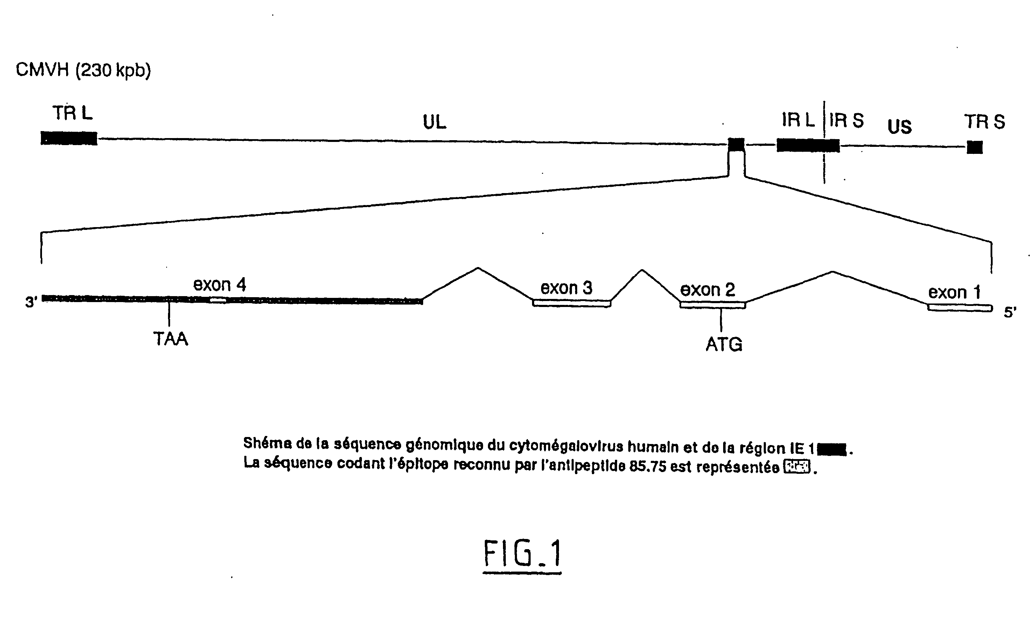

Figure 1 : ***Représentation schématique du fragment d'ADN génomique viral contenant l'ADNc de IE1.***

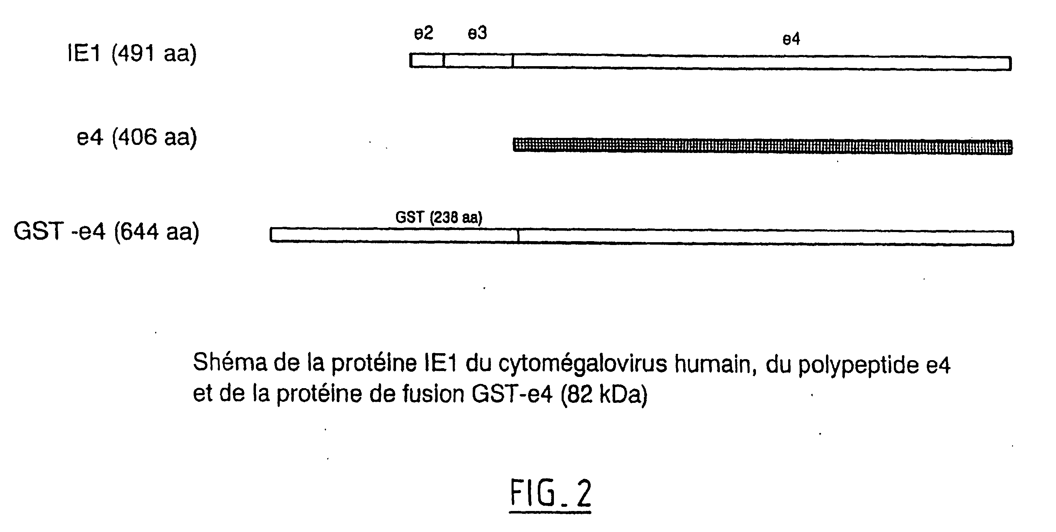

Figure 2 : ***Représentation schématique de la protéine de fusion GST-e4***

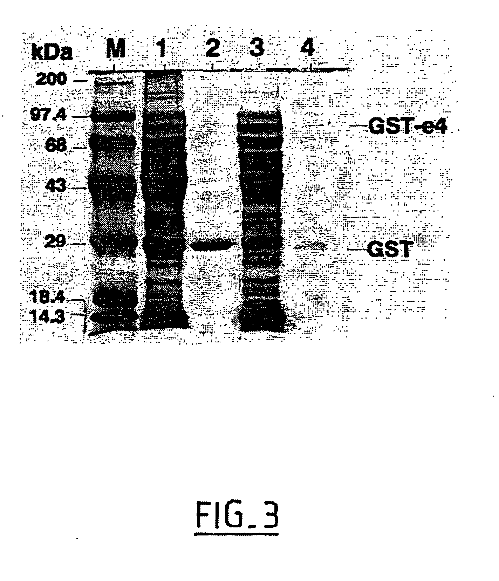

Figure 3 : ***Analyse de l'expression de GST-e4 par E. Coli en électrophorèse sur gel de polyacrylamide-SDS (SDS-PAGE)*.:** Les lysats de bactéries non recombinantes (1) et recombinantes GST-e4 (2) ont été soumis à une SDS-PAGE et colorés au bleu de coomassie. Après passage sur colonne d'affinité-glutathion, les éluats ont été analysés dans les mêmes conditions (2, 4). Le poids moléculaire apparent des marqueurs (M) est indiqué.

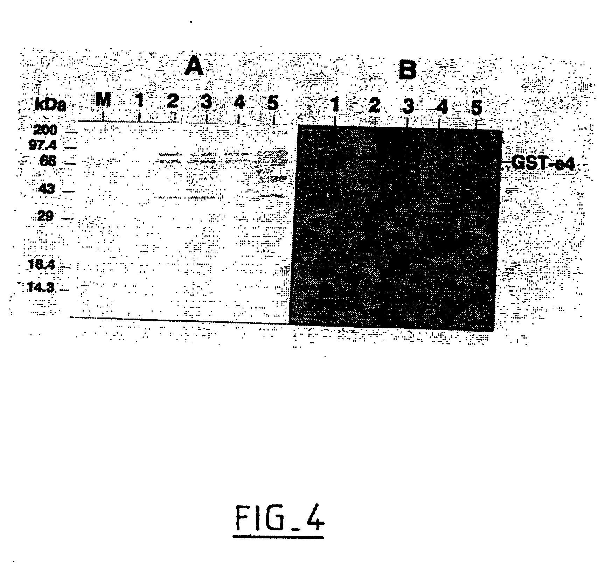

Figure 4 : ***Analyse en "western blot" (A) et autoradiographie (B) de l'expression de GST-e4 par E. Coli*.** Les lysats bactériens ont été soumis à une SDS-PAGE puis transférés sur membrane de nitrocellulose. Une incubation de la membrane en présence de l'anti peptide 85.75 a révèlé la présence de GST-e4 dans le lysat total (5), le culot et le surnageant de sonication (4, 3), et dans la fraction retenue sur la colonne d'affinité (2). La piste 1 contient un lysat de bactéries non recombinantes. La figure 4.B correspond à l'autoradiographie de la membrane.

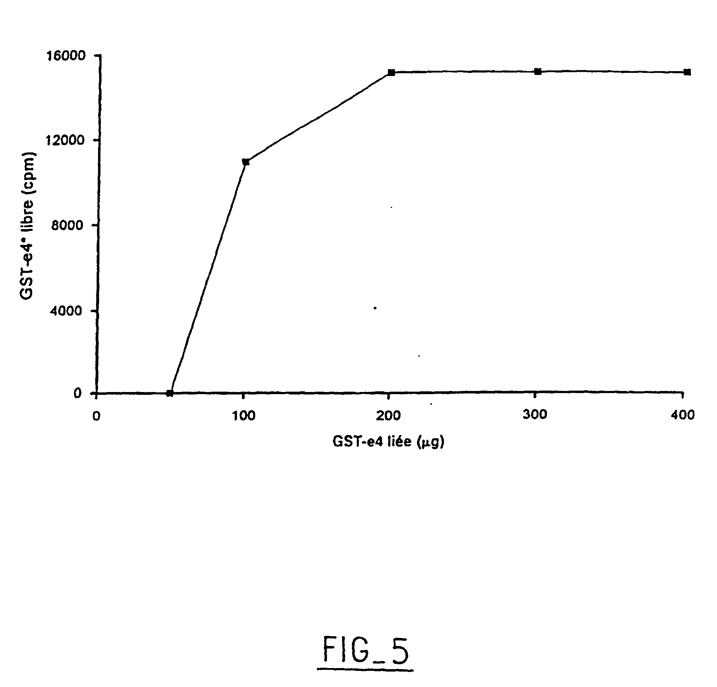

Figure 5 : ***Courbe de saturation des BVSM par GST-e4 purifiée:*** Des quantités croissantes de GST-e4 froide (50 à 400 µg) ont été piéincubées avec des BVSM (100 µg). Les complexes ainsi formés, ont été incubés en présence de GST-e4 radiomarquée (GST-e4*) et le taux d'association (cpm) de la protéine chaude a été déterminé par comptage après ultrafiltration sur Microsep 300.

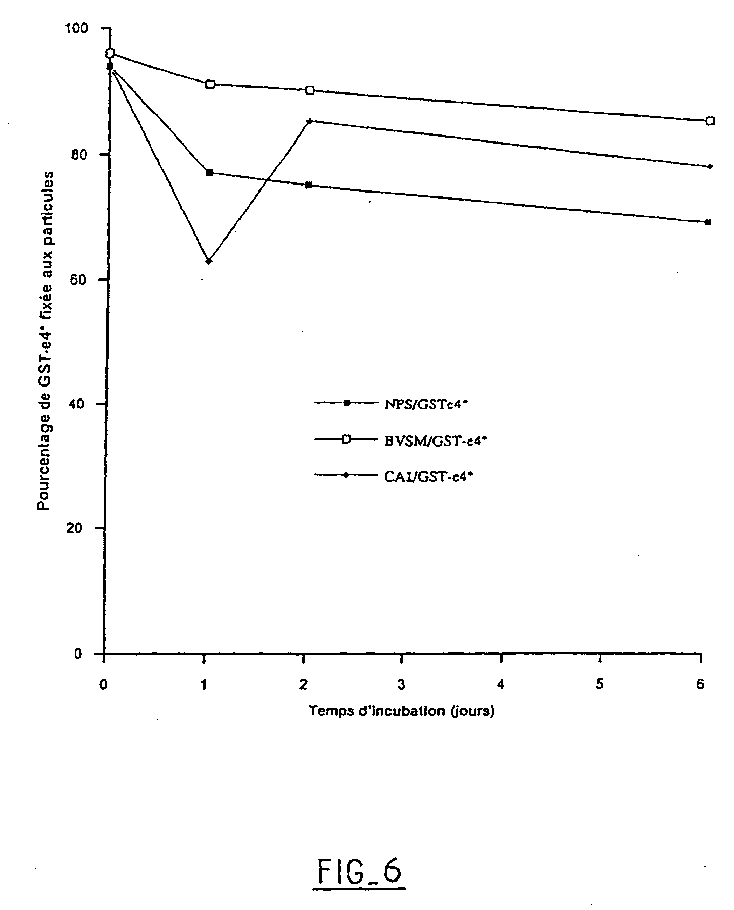

Figure 6 **:*Stabilité de l'association GST-e4***/ ***particule:*** Des complexes GST-e4*/particule ont été incubés en milieu RPMI/SVF à 37°C de 0 à 6 jours. Le pourcentage de GST-e4* associé au terme de chaque période d'incubation a été déterminé par comptage après ultrafiltration.

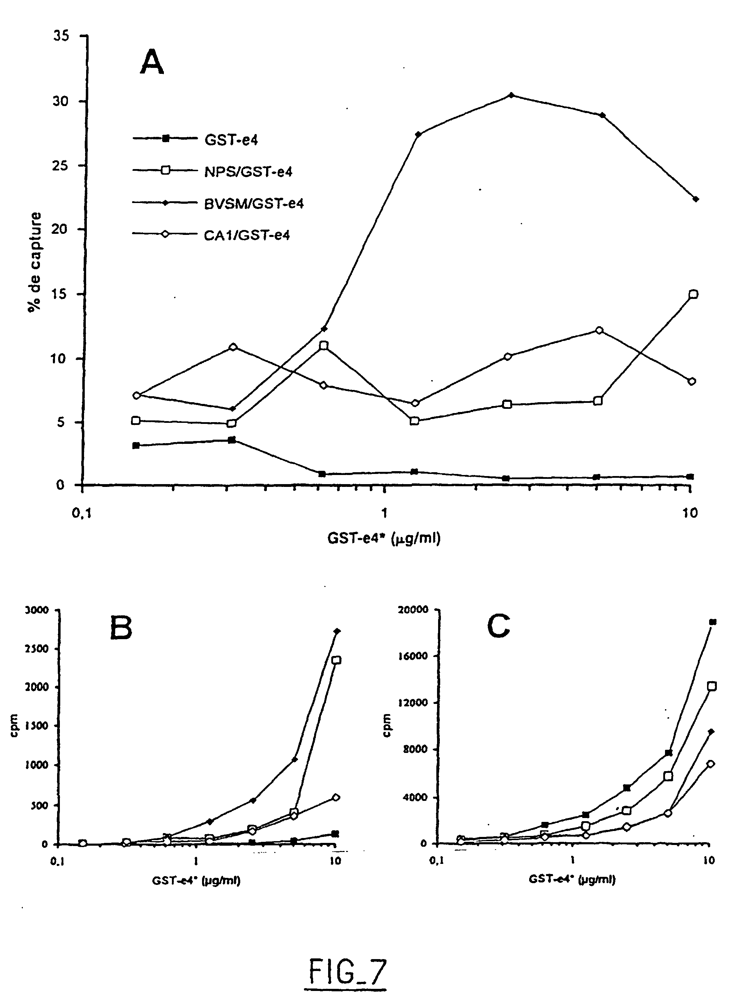

Figure 7 : ***Capture de GST-e4* soluble ou associée aux particules par des lymphocytes B*./*EBV*** L'antigène radioactif GST-e4*, libre ou associé à des particules (NPS, CA, BVSM) a été incubé en présence de 5x10⁵ lymphocytes B/EBV dans du RPMI/SVF, à 37°C pendant 15h. Le pourcentage de capture (A), la quantité d'antigène associé aux cellules (B), et la quantité d'antigène retrouvé dans le milieu (C) sont représentés en fonction de la concentration en protéine dans les puits

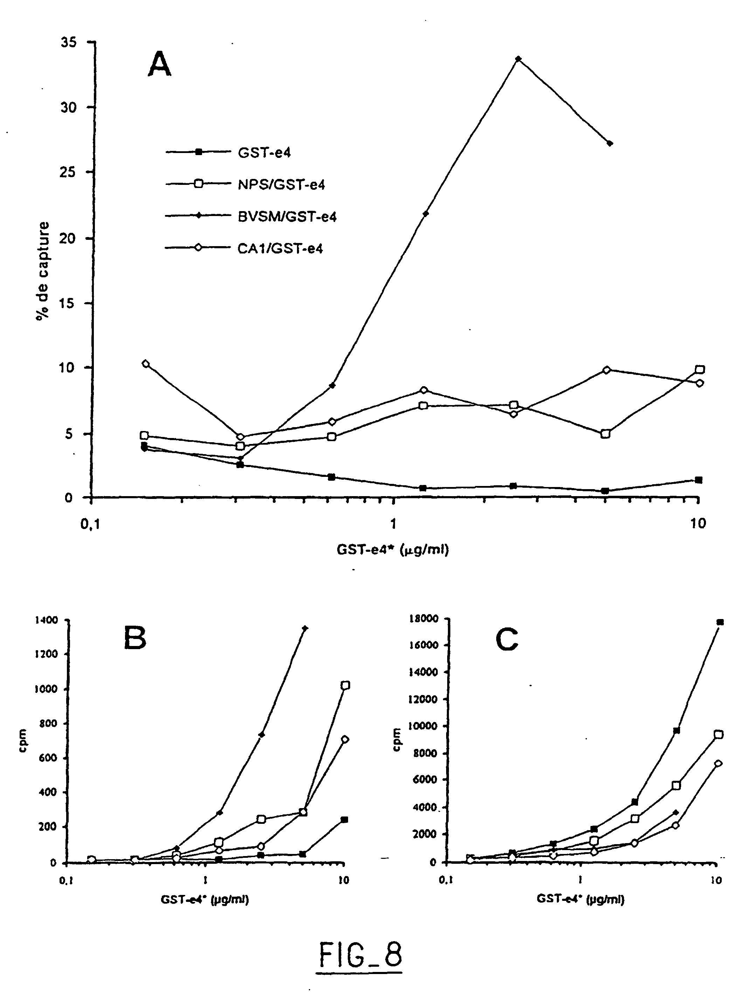

Figure 8 : ***Capture de GST-e4* soluble ou associé aux particules par la lignée macrophagique "Mono Mac 6":*** L'antigène radioactif GST-e4*, libre ou associé à des particules (NPS, CA, BVSM) a été incubé en présence de 5x10⁵ cellules Mono Mac 6 dans du RPMI/SVF, à 37°C pendant 15h. Le pourcentage de capturé (A), la quantité d'antigène associé aux cellules (B), et la quantité d'antigène retrouvée dans le milieu (C) sont représentés en fonction de la concentration en protéine dans les puits

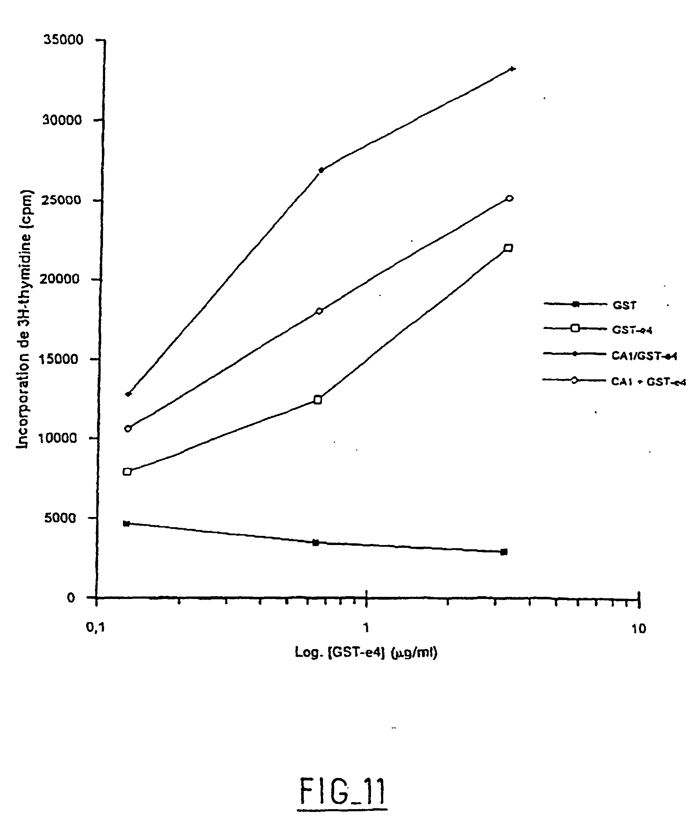

Figure 9 : ***Test de prolifération du clone (A3) en présence de lymphocytes B*/*EBV et du mélange CA vides*/*GST-e4:*** Les cellules du clone A3 ont été incubées en présence de lymphocytes B/EBV irradiés et d'antigène soluble seul (GST, GST-e4), ou avec des CA vides (CA + GST-e4), et avec l'antigène associé à des CA (CA/GST-e4). La prolifération a été déterminée par l'incorporation de [³HJ-thymidine, mesurée en cpm.

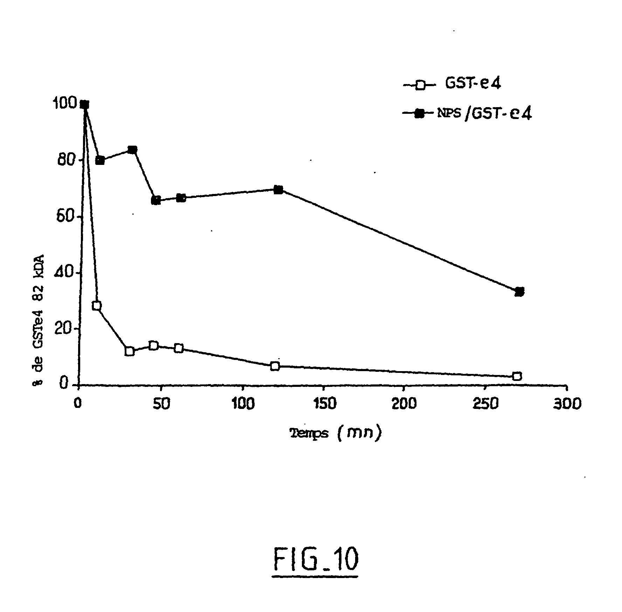

Figure 10 : le GST-e4 est protégé de la protéolyse grâce à sa fixation à des biovecteurs. Du GST-e4, libre ou associé à des NPS, a été incubé avec de la trypsine (rapport en masse 1/600), et on a mis fin à la réaction comme indiqué. Des échantillons ont été soumis à une SDS-PAGE, et le GST-e4 de 82 kDa a été quantifié par exploration densitométrique du gel. Les résultats ont été exprimés en pourcentage du GST-e4 de départ.

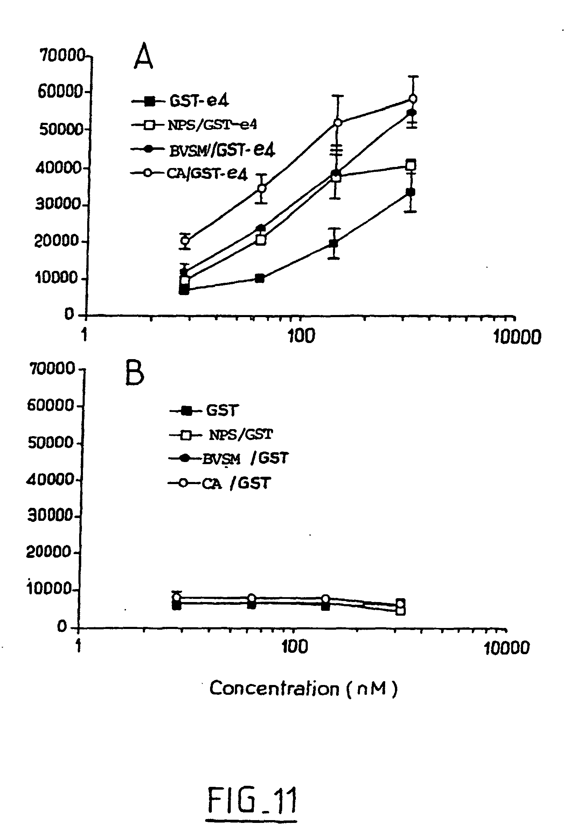

Figure 11 : La réponse des lymphocytes T CD4+ au GST-e4, est améliorée in vitro après fixation aux biovecteurs avec des PBL comme CPA. Les cellules provenant du clone "BeA3" ont été cultivées en présence de PBL irradiés et de différentes concentrations de GST-e4 (A) et de GST (B), sous forme libre ou particulaire.

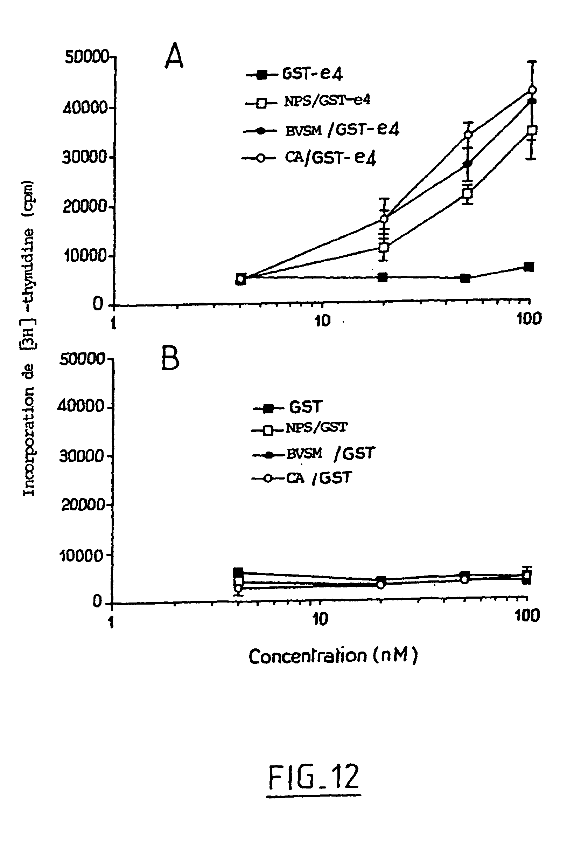

Figure 12 : On a amélioré in vitro la réponse des lymphocytes T CD4+ au GST-e4 après fixation à des biovecteurs avec des lymphocytes B transformés par le virus EBV, à titre de CPA. Les cellules provenant du clone "BeA3" ont été cultivées en présence de cellules B transformées par l'EBV et irradiées, et de différentes concentrations de GST-e4 (A) et de GST (B), sous forme libre ou particulaire.

### Exemples

### 1) Matériels et méthodes

### Synthèse des vecteurs canonisés de taille 80 nm :

Les vecteurs utilisés dans cette étude ont tous été dérivés du même noyau polysaccharidique synthétisé à partir du même lot (NPS). Ces NPS ont été soit acylés avec de l'acide palmitique (CA), soit phospholipidés avec différents types de phospholipides (BVSM).

### Synthèse des NPS

100 g de Maltodextrine (Glucidex 6, Roquettes), conforme à la monographie maltodextrine de la pharmacopée US NF XVII, ont été dissous dans 17 ml d'eau sous agitation mécanique violente puis transférés dans un réacteur en verre, auquel ont alors été ajoutés 2.4 g de NaBH₄. Une demi heure plus tard, ont été ajoutés 44 ml de soude 10 N puis 9.72 ml (0.124 mole) d'un agent réticulant, l'épichlorhydrine (Fluka). Après 90 min de réaction à température ambiante, 31.18 g d'un agent cationisant, l'hydroxycholine (glycidyltrimethylammonium chloride, Fluka), préalablement dissout dans 20 ml d'eau ont été ajoutés. L'incubation a été maintenue pendant 20 h sous agitation mécanique. Le gel obtenu a été dilué avec 1.5 1 d'eau puis neutralisé à l'acide acétique. Le gel a ensuite été broyé dans un homogénéisateur par extrusion sous haute pression, puis ultrafiltré sur module filtrant de 40 kDa. L'ultrafiltration a été stoppée lorsque l'ultrafiltrat ne réagissait plus au nitrate d'argent. L'analyse de la taille des nanoparticules (NPS) a été effectuée avec un Nanosizer Coulter N4SD.

### Synthèse des coeurs acylés (CA)

A 10 g de NPS fraichement Lyophilisés préalablement dispersés dans 100 ml de dichlorométhane (distillé extemporanément), ont été ajoutés 9,34 ml de chlorure d'acide palmitique. La réaction a été poursuivie pendant 4 heures à température ambiante. Après avoir ajouté 200 ml de diéthyl éther au milieu réactionel, les CA ont été filtrés sur Buchner, lavés à l'éthanol, repris dans 500 ml d'eau et homogénéisés.

### Synthèse des BVSM

Les NPS ont été phospholipidés (20% pds/pds de NPS lyophilisés) avec différentes proportions de phospholipides

### Tests de toxicité in vivo

Les vecteurs testés (NPS, CA, BVSM) ont été remis en suspension dans l'eau, à une concentration de 1mg/ml. La pyrogénicité des échantillons a été déterminée *in vivo* chez le lapin à la dose de 100 µg de vecteur par dose selon la pharmacopée Européenne.

### Production de la protéine de fusion GST-e4 dans Escherichia coli :

### Clonage dans Escherichia coli:

L'ADNc correspondant à l'exon 4 (e4, figure 1) a été obtenu par amplification (PCR) à la Taq polymérase (Proméga, Coger, France) à partir du plasmide pRL 103 (don de R. LaFemina, (9)) contenant la région IE du CMV (souche Towne). Les amorces, A 1 (A1 = 5'CCCGGGℓAATTCTCATGGTCAAACAGATTAAGGTTCGAG3') et A2 (A2=. 5'CCCGGGA 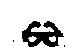 AGCTTTTACTGGTCAGCCTTGCTTCTA3') correspondant respectivement à l'extrémité 5' et 3' de e4 ont été utilisées. Des sites EcoR I (ℓ) et Hind III ( 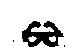 ) ont été introduits dans ces amorces pour permettre l'insertion du fragment de PCR dans le plasmide pGEX-KG (10). Le fragment inséré est en phase avec le gène de la glutathion-S-transférase (GST) dont le promoteur est inductible à l'isopropyl-thiogalactoside (IPTG, Sigma, France). Entre l'extrémité 3' du gène de la GST et le site multiple de clonage se situe une région codant pour un site de clivage peptidique spécifique de la thrombine. La transformation des bactéries *Escherichia coli* DH5α (Gibco, Cergy, France) et le criblage des recombinants ont été effectués selon les techniques classiques (11). L'insertion du produit de PCR correspondant à l'exon 4 dans pGEX-KG a été vérifiée par l'analyse, sur plusieurs clones, des fragments de restriction de l'ADN plasmidique, visualisés après électrophorèse en gel d'agarose à 1% en bromure d'éthidium (BET). L'insertion dans pGEX-KG a été confirmée en testant l'expression du produit recombinant GST-e4 après induction par l'IPTG. Les clones positifs ont été séquencés selon la technique de Sanger (12) avec un kit *"Hot tub sequenase*" (Pharmacia, Saint Quentin Yvelines, France) selon les indications du fournisseur.

### Expression et purification de GST-e4 :

Les bactéries recombinantes conservées à -80°C ont été mises en culture dans du milieu "Luria broth" (Difco, Osi, France) contenant 50 µg/ml d'ampicilline (LB/Ap) (Sigma). Cette préculture, diluée au 1/10ème en LB/Ap a été cultivée dans les mêmes conditions jusqu'à DO/600nm = 1.0. L'induction de l'expression du produit recombinant GST-e4 a été réalisée en ajoutant 100 µM d'IPTG à la culture. L'incubation a été poursuivie pendant 2 heures à 25°C pour éviter la formation de corps d'inclusions (13). Les bactéries, récoltées par centrifugation ont été lavées dans du PBS ("phosphate buffered saline", pH 7.3, 4.3 mM Na₂HPO4, 1.4 mM KH₂PO4, 137 mM NaCl, 2.7 mM KCl). Le culot de cellules, conservé une nuit à -80°C puis décongelé a été repris dans 50 ml de tampon de lyse (PBS, 1mg/ml lysozyme, 1mM phényl-méthyl-sulfonide-fluoride (PMSF) ). La suspension a été incubée pendant 30 minutes dans la glace puis soniquée. Le lysat a été agité pendant 30 minutes à température ambiante en présence de TRITON X 100 (concentration finale:1%) puis clarifié par centrifugation (30000g/1h). Le surnageant a été déposé sur une colonne d'affinité greffée avec le substrat de la GST (14) (Glutathione/Sepharose 4B, Pharmacia) préalablement équilibrée dans du PBS. Après lavage de la colonne avec du PBS, la protéine de fusion GST-e4 a été éluée avec 10 mM de glutathion réduit dans le tampon d'élution 50 mM Tris HCl, pH 8.0.
Des marquages métaboliques de GST-e4 ont été réalisés en ajoutant au milieu de culture et à raison de 10 µCi/ml, les résidus L-[³⁵S]-Methionine et -Cystéine (³⁵S Express, NEN, Les Ulis, France) au moment de l'induction par l'IPTG. La purification du produit radioactif (GST-e4*) a été réalisée dans les mêmes conditions que pour le produit non radiomarqué. La concentration des produits purifiés a été déterminée par la méthode de Bradford (Kit "*Bio-Rad Protein assay",* Biorad, Yvry sur Seine, France). La production de la protéine de fusion GST-e4 a été contrôlée par électrophorèse en gel de polyacrylamide à 10% en présence de SDS (SDS PAGE) (15). Des marqueurs de poids moléculaire précolorés (Gibco) ont été utilisés. La révélation a été effectuée soit par coloration du gel au bleu de Coomassie soit en *"western blot"* (16) après transfert sur membrane de nitrocellul (Hybond C super, Amersham, Les Ulis, France) comme suit. Après saturation dans du "blotto" (PBS, 0.2% NP40, 2.5% lait écrémé), la membrane a été incubée pendant 1 heure avec l'immun sérum d'un individu séropositif pour le CMV (CMV+) ou avec l'antipeptide de lapin 85,75 reconnaissant un épitope situé dans la partie C-terminale de l'exon 4 (don de J. A Nelson, (17)). L'immunsérum et l'antipeptide ont été dilués respectivement au 1/100^{ème} et au 1/500^{ème} dans du "blotto". La membrane a été lavée 3 x 5 minutes dans du PBS et incubée pendant 1 heure avec des IgG de chèvre anti-Ig humaines ou anti-Ig de lapin marquées à la peroxydase (GAH/PO, GAR/PO, Nordic, Tebu, Le Perrey en Yvelines, France) diluées au 1/500^{ème} dans du "blotto". Après 3 lavages de 5 minutes chacun dans du PBS, la membrane a été incubée dans la solution de révélation (PBS, 0.5 mg/ml diaminobenzidine (DAB), 0.1% H₂O₂). Quand les produits analysés étaient radiomarqués, une autoradiographie du gel ou de la membrane après *western blotting* a été réalisée sur film Kodak X-OMAT (Polylabo, France).

### Association de GST-e4* aux particules :

La protéine de fusion GST-e4*, en solution dans du tampon d'élution 50 mM Tris-Cl, pH 8.0, a été incubée avec les trois principaux types de particules (NPS, CA, BVSM) en suspension dans l'eau, à une concentration de 1mg/ml. L'incubation a été effectuée à 4°C sous agitation pendant 1 heure. Le pourcentage d'association a été déterminé en mesurant la radioactivité associée aux particules. L'antigène libre a été éliminé par ultrafiltration à 5000g sur une membrane ayant un seuil de coupure de 300 kDa (microsep 300, Filtron, Coignieres, France). La mesure de la radioactivité libre dans l'éluat, dans les lavages et associée aux particules a été réalisée trois fois sur un compteur β (BETAmatic, Kontron). Le pourcentage d'association a été calculé en faisant le rapport de la radioactivité liée sur la somme de la radioactivité libre et de la radioactivité liée aux particules. Des expériences d'association de GST aux particules ont été réalisées dans les mêmes conditions qu'avec GST-e4*. L'association de la GST aux panicules après lavage a été confirmée en SDS PAGE

### Stabilité de GST-e4* associée aux particules :

L'association de GST-e4* aux trois types de particules a été effectuée comme précédemment avec un rapport GST-e4*/particule de 0.1 (R=0.1). Les lavages ont été réalisés en RPMI/10% sérum de veau foetal (RPMI/SVF) (Gibco) supplémenté avec 1 mM de pyruvate de sodium , 2mM de L-glutamine, 200 U/ml de pénicilline et 80 ng/ml de streptomycine. Les complexes GST-e4*/particule ont été repris dans le même milieu et incubés à 37°C. Des aliquots ont été prélevés à différents temps, et le pourcentage de GST-e4* associé aux particules a été déterminé comme précédemment.

### Capture de GS7-c4* par des lignées de lymphocytes B et de macrophages "in vitro":

L'analyse de la capture de GST-e4* soluble ou associé aux particules a été effectuée sur des lignées de macrophages ("MonoMac 6", DSM ACC 124) et de lymphocytes B immortalisés par le virus d'Epstein Barr (B/EBV). Les cellules (5x10⁵/ml), en plaques de 24 puits à fonds plats (Falcon, Becton Dickinson, New Jersey), ont été incubées dans du RPMI/SVF en atmosphère humide à 5% de CO₂ , avec différentes concentrations de GST-e4* soluble ou complexé aux particules (R=0.1). Après 15 heures d'incubation, les cellules ont été récoltées et lavées 2 fois dans du PBS. La radioactivité libre a été comptée dans le surnageant, dans le liquide de lavage et dans les cellules resuspendues dans du PBS. Les résultats de comptage correspondent à la moyenne de trois mesures. Le pourcentage de capture pour une concentration antigénique donnée a été exprimé par le rapport de la radioactivité liée aux cellules sur la radioactivité totale.

### Digestion trypsique d'un antigène associé à des nanoparticules

On a incubé à la température ambiante du [35S] GST-e4 soluble, et des mélanges de l'antigène avec différentes nanoparticules, avec de la trypsine provenant du pancréas de boeuf (Sigma), le rapport en masse de l'enzyme au substrat étant de 1:600. Au bout de différents intervalles de temps, on a mis fin aux réactions (aliquotes de 20 µl) par congélation immédiate dans de l'azote liquide. Avant SDS-PAGE, les échantillons ont été décongelés et portés à ébullition dans un tampon échantillon de Laemmli pendant 5 minutes. Pour vérifier que les particules n'inhibaient pas l'activité protéolytique de la trypsine, on a incubé des mélanges contenant 5 µg de l'enzyme, en présence ou en l'absence de 3 mg de particules par ml, avec le substrat enzymatique Chromozym-Try (Boehringer Manheim, France), selon les instructions du fabricant. On a déterminé l'activité de trypsine par l'augmentation d'absorbance à 1 = 405 nm. On a quantifié la protéine GST-e4 de 82 kDa par exploration densitométrique du gel coloré au Coomassie.

### Analyses par immunofluorescence et par microscopie confocale

On a utilisé des lymphocytes B transformées par l'EBV provenant du donneur "Be" et la lignée lymphocytaire B Raji du lymphome de Burkitt. On a incubé les cellules dans un milieu RPMI sans sérum complété par un tampon HEPES 20 mM pH 7,2 et 1 mg/ml de sérumalbumine de boeuf (BSA), à 37°C ou à 4°C, avec de la rhodamine-transferrine 600 nM et/ou 45 mg/ml de FITC-CA, pour différents temps d'incubation. Puis on a lavé les cellules à deux repri-ses dans du PBS froid contenant 1 mg/ml de BSA (BSA-PBS), une fois dans du PBS froid seul, et on les a fixées pendant 30 minutes à la température ambiante dans du PBS contenant 3,7 % de paraformaldéhyde et du saccharose 30 mM. Après 10 minutes d'incubation dans du NH₄Cl-PBS 50 mM, trois lavages dans du BSA-PBS et un lavage dans le PBS, on a monté les cellules sur des lamelles de microscope dans 25 mg/ml de Dabco (1,4-diacylbicyclo-(2.2.2)octane, Sigma St. Louis, MO), 100 mg/ml de mowiol (Calbiochem, La Jolla, CA), 25 % (v/v) de glycérol, Tris-HCl 100 mM pH 8,5. Les échantillons ont été examinés au microscope confocal (Leica) fixé à un microscope diaplan (Leitz) équipé d'un double laser, argon-krypton. Des coupes optiques en série ont été enregistrées à des intervalles de 0,5 mm à l'aide d'un objectif de 63x. Les photographies ont été prises sur une pellicule Ilford XP2 400 ASA.

### Tests de prolifération des clones TCD4+ anti IE1:

L'obtention des clones de lymphocytes TCD4+ anti IE1 et l'analyse de leur prolifération en présence de cellules présentatrices d'antigène (CPA) autologues de donneurs CMV+ (Centre de Transfusion Sanguine de Toulouse) ont été décrits précédemment (18). Brièvement, 2x10⁴ cellules d'un clone T CD4⁺ ont été incubées dans des plaques de 96 puits à fonds ronds (Falcon) avec les CPA irradiées correspondant à 1x10⁵ leucocytes du sang périphérique (PBL) ou 5x10⁴ lymphocytes B/EBV, autologues ou possédant des molécules du CMH de classe II de même allotype DR. Les cellules ont été incubées dans un volume final de 100 µl en RPMI contenant 10% d'un pool de sérums humains (RPMI/SH) (Laboratoires FANDRE, Ludres, France) et différentes dilutions d'antigène libre ou associé aux particules. Alternativement, l'Ag soluble et les particules vides ont été ajoutés extemporanément aux cellules. Les complexes Ag/ particule ont été stérilisés par irradiation. Trois jours plus tard, la culture a été marquée pendant 15 heures avec 1 µCi par puits de [³H]-thymidine (49 Ci/mmole, Amersham).

La prolifération des clones T est déterminée par la mesure de l'incorporation de [³H]-thymidine par les cellules. Les résultats exprimés en cpm correspondent à la moyenne de trois expériences concomitantes.

Les extraits d'astrocytomes humains (U373MG) transfectés par l'ADN génomique lE (A2) ou non transfectés (A0) qui avaient été utilisés pour générer les clones (18), ont été employés comme contrôles positifs et négatifs, respectivement. La détermination des allotypes DR des différents donneurs a été effectuée par PCR avec des amorces oligonucléotidiques spécifiques (Pr Abbal, Lab. Centr. Immunol, CHU Rangueil, Toulouse). Les clones D11 et A3 provenant d'individus (DR3/DR4) et (UR8/DR7) respectivement, ont été utilisés.

### 2) Résultats

### Caractérisation des particules synthétisées

La taille des particules étaient de 80 nm, avec une déviation standart de 20 nm. Le taux de charge a été titré à 0,8 mmole d'hydroxycholine par gramme de nanoparticules lyophilisées. Les échantillons testés étaient apyrogènes.

### Production de GST-e4

### Expression de GST-e4 dans E Coli

L'expression du produit de fusion GST-e4 dans les lysats bactériens a été testée en SDS-PAGE. Les figures 3 et 4 correspondent à l'analyse des lysats de bactéries cultivées en présence d'IPTG. La figure 3 a révèlé la présence après coloration au bleu de coomassie, d'une bande correspondant à une masse relative de 82000 dont l'intensité est plus importante dans le lysat de bactéries recombinantes (piste 3) que dans l'échantillon de bactéries non recombinantes (piste 1). En revanche, dans la piste 1 une production importante de GST (Mr 27000) a été mise en évidence. L'analyse en *" western blotting"* des mêmes échantillons avec l'asti peptide 85.75 (Fig 4A) et un immunsérum humain anti-CMV a révélé une réactivité spécifique associée à une protéine de masse relative 82000 en accord avec la masse attendue pour la protéine de fusion GST-e4 (Figure 2). L'autoradiographie de la membrane (Fig 4B) a révélé une bonne incorporation des résidus radiomarqués dans la protéine de 82 kDa révélée par l'anti peptide, dans les conditions de culture décrites précédemment.

### Purification de GST-e4 par chromatographie d'affinité

Les lysats bactériens ont été soumis à une chromatographie sur sépharose-glutathion et anlysés en SDS-PAGE. Les figures 3 et 4 montrent que les protéines GST (piste 2, Mr 27000) et GST-e4 (piste 4, Mr 82000) ont été retenues spécifiquement par le support. Des bandes de masse plus faible co-migrant avec GST-e4 ont été visualisées après *western blotting* (Fig4A). Ces produits copurifiés avec GST-e4 résultent probablement de la dégradation de la protéine de fusion et contiennent la séquence peptidique reconnue par l'antisérum 85.75. Aucun signal n'a été observé dans la piste correspondant à la GST (piste 1). Nous avons obtenu de façon reproductible et après un seul passage sur la colonne 1mg de GST-e4* avec une activité spécifique de 400mCi /mmole.

### Association de GST-e4* aux particules

### Rendement d'association en fonction du type de particules

Les particules (NPS, CA, BVSM) ont été incubées en présence de GST-e4 radiomarquée (GST-e4*) à raison de 1mg de protéine pour 10 mg de vecteur (R=0.1). Les taux d'association moyens (20 tests) sont de l'ordre de 90% et identiques quelles que soient les particules.

### Saturation des particules par GST-e4*

Des BVSM (100 µg) ont été préincubés avec des quantités croissantes de GST-e4 froide dans un rapport de masse Ag/BVSM allant de 0.1 à 4 (R=0.1 à R=4). Pour chacun de ces rapports, GST-e4* a été ajoutée aux complexes et le taux d'association de la protéine chaude aux vecteurs a été déterminé par comptage après ultrafiltration. La figure 5 montre que les BVSM sont saturés en antigène quand le rapport massique Ag/BVSM est égal à 2.

### Stabilité de l'association GST-e4*/ particules en fonction du temps

Des complexes GST-e4/particule (R=0.1) ont été incubés en RPMI/SVF à 37°C. L'analyse du pourcentage de GST-e4 qui est resté associé aux particules après plusieurs jours d'incubation (Fig 6), a montré que les cinétiques de relargage sont identiques pour les trois types de particules, avec une pente plus forte sur les premières 24 heures. Après 6 jours d'incubation, le taux de GST-e4* associé est de 85%, 80%, et 70% avec les BVSM, CA, et NPS, respectivement. Une analyse en SDS-PAGE des mêmes échantillons suivie d'une autoradiographie a permis de visualiser la protéine associée aux particules.

### L'antigène a été associé d'une manière stable à des vecteurs, et protégé d'une protéolyse

Le rendement d'association du [35S]-GST-e4 était compris entre 80 et 90 % et ne dépendait pas de la nature des particules cationisées (NPS, CA, BVSM), et, quand l'antigène a été incubé avec des vecteurs anioniques (CA phosphaté), il n'y a eu piégeage d'aucune substance, comme on pouvait s'y attendre d'après le point isoélectrique acide du GST-e4. Ce résultat a donné à penser que la nature des interactions entre l'antigène et les particules était essentiellement ionique. Pour analyser la stabilité de l'association du GST-e4 aux vecteurs, on a déterminé en fonction du temps la libération de l'antigène à partir des particules dans les conditions de culture. Sur 6 jours d'incubation dans un milieu complet (10 % SVF), la cinétique de la libération de l'antigène était identique pour le NPS, pour le CA et pour le BVSM, et la libération n'a jamais dépassé 30 % pour le NPS, 20 % pour le CA et 15 % pour le BVSM. Nous avons déterminé si l'antigène était protégé d'une protéolyse par son association à des vecteurs. Dans ce but, on a soumis un antigène [35S]-GST-e4, soluble et complexé, à une digestion par la trypsine, et on l'a analysé en SDS-PAGE. Le gel a ensuite été exploré pour déterminer la présence du produit de 82 kDa. Une exploration densitométrique a montré que, en deux heures d'incubation avec l'enzyme, 30 % de l'antigène complexé étaient accessibles à la protéolyse, alors que 85 % de l'antigène sous forme soluble étaient dégradés (Figure 10). L'effet protecteur n'était pas dû à une inactivation directe de l'enzyme par la particule, car la cinétique de l'activité enzymatique par utilisation du substrat "chromozyme Try" n'était pas affectée par la présence de vecteurs.

### Capture de GST-e4* par des lignées de lymphocytes B et de macrophages

Des complexes GST-e4*/particule (R=0.1) et la protéine soluble GST-e4* ont été incubés en quantité croissante avec des lignées de macrophages et de lymphocytes B/EBV. Une cinétique d'association des préparations d'antigène (0.6 µg/ml, R=0.1) aux cellules a montré une incorporation équivalente, pour un type d'échantillon donné, et pour des temps d'incubation allant de 10 min à 15h (résultat non montré). Les figures 7 et 8 représentent le pourcentage d'antigène associé aux cellules après 15h d'incubation, en fonction de la quantité de protéine complexée ou soluble ajoutée. Ces figures montrent que pour les lymphocytes B/EBV (Fig 7) et les macrophages (Fig 8), la quantité d'antigène soluble associée aux cellules est très faible même à des concentrations élevées. En ce qui concerne la capture de l'antigène en fonction des différents types de particule, les BVSM ont permis d'obtenir le rendement d'incorporation le plus élevé (30%) avec les deux types cellulaires. Des rendements de l'ordre de 5% à 10%, ont été obtenus avec les NPS et les CA, alors qu'ils avoisinent 1% avec l'antigène soluble.

### Des vecteurs synthétiques sont internalisés par des cellules présentatrices d'antigènes (CPA)

Pour étudier plus en détail le mécanisme par lequel les particules assurent la médiation de l'augmentation de la capture de l'antigène par les cellules, nous avons analysé leur aptitude à être internalisé par les CPA par utilisation d'une microscopie confocale. On a incubé des lymphocytes B transformés par l'EBV et provenant de donneur "Be", avec des particules de CA marquées à la fluorescéine, et de la rhodamine-transferrine, pendant différents laps de temps à une température de 4° C ou de 37°C. Puis les cellules ont été fixées, montées et observées sous un microscope confocal, comme décrit dans Matériels et Méthodes. On a procédé à une série Z de coupes optiques selon des incréments de 0,5 µm. On a obtenu d'une manière séquentielle les résultats de la mesure des émissions de la fluorescéine et de la rhodamine, à partir de la même cellule. Chaque image présente une coupe optique médiale d'une cellule représentative : fluorescéine ou rhodamine. Barre : 10 nm.

Dans ce but, on a incubé pendant 19 heures à 37°C, avec des particules de CA marquées à la fluorescéine, des lymphocytes B transformés par l'EBV et provenant du donneur "Be". Des particules de CA ont été trouvées dans les vésicules intracellulaires, ce qui montre qu'elles se trouvent en fait à l'intérieur des cellules. Pour étudier plus en détail le mécanisme responsable de l'internalisation des vecteurs synthétiques, nous avons étudié la cinétique de l'entrée, et nous l'avons comparée à celle de la transferrine, utilisée comme marqueur pour l'endocytose médiée par un récepteur. Les cellules ont rapidement internalisé la transferrine, ce qu'on voit facilement dans les vésicules intracellulaires. Par contraste, les vecteurs fluorescents CA, BVSM ou NPS ont été rarement observés à l'intérieur des cellules, pour des temps d'incubation inférieurs à 3 heures. Au contraire de la transferrine, les particules fluorescentes se sont accumulées sous forme de taches ou de capuchons de grandes dimensions sur la surface des cellules. Au bout de 5, 7 et 19 heures à 37°C, la plupart des cellules présentaient des particules de CA dans les compartiments intracellulaires, bien que des organelles cellulaires se révélassent différentes de celles qui contenaient la transferrine internalisée. On a obtenu des résultats analogues en étudiant des cellules provenant de la lignée cellulaire B Raji du lymphome de Burkitt.

Nos résultats ont montré que les vecteurs CA ont été internalisés par les CPA, en s'accumulant lentement dans les compartiments intracellulaires. De plus, tant la cinétique d'entrée que leur localisation intracellulaire ont donné à penser que ces particules suivaient une voie d'endocytose différente de celle de la transferrine.

### Une réponse spécifique des cellules T au CD4+ est renforcée in vitro après association du GST-e4 à des nanoparticules

Nous avons étudié si la protéine de fusion recombinée GST-e4, libre ou associée à des particules, pourrait être efficacement présentée par des CPA à des clones de lymphocytes T de CD4+ anti IE1, restreintes à DR3 et DR8. Les résultats d'une réponse proliférative représentative du clone BeA3, en présence de PBL apparié en HLA (Figure 11) ou de lymphocytes B (Figure 12) sont présentés. On a obtenu des résultats analogues avec le clone FzD11. Aucune prolifération de cellules T n'a été observée quand on incubait des particules vides, ou des particules associées à GST, en présence ou en l'absence de PBL (Figure 11B) et de lymphocytes B (Figure 12B), Par contraste, la capacité du GST-e4 particulaire à stimuler la prolifération des clones a été renforcée avec tous les types de vecteurs. Quand on utilisait des PBL comme CPA, cet effet adjuvant allait de 5 à 25 fois. De façon remarquable, on avait besoin d'une quantité jusqu'à 25 fois plus faible de protéines sous forme complexée (CA), pour induire une réponse proliférative d'intensité analogue à celle que l'on obtient avec l'antigène libre (Figure 11A). Quand on a utilisé des lymphocytes B pour présenter l'antigène particulaire (Figure 12A), l'effet a été du même ordre de grandeur que celui observé avec les PBL. Pour déterminer si la liaison de l'antigène à la particule était une condition préalable à cet effet, on a ajouté extemporanément aux cellules des CA vides et l'antigène soluble. Dans ces conditions, la potentialisation a encore été observée, mais beaucoup plus faible (2 fois), ce qui pourrait être attribué à une association rapide de l'antigène aux vecteurs au moment de leur introduction dans les puits de culture, car la fixation totale du GST-e4 aux particules était réalisée en 1 heure à 4°C (Matériels et Méthodes).

### Bibliographie

(1) Alford C.A., and Britt W.J. (1990) Cytomegalovirus. In: *Virology*, Second Edition, (ed. B. N. Fields, and D. M. Knipe et al. ), Raven Press Ltd., New York.
(2) Rasmussen, L (1993) Progress in the development of a human Cytomegalovirus glycoprotein subunit vaccine. In S Michelson, SA Plotkin eds. Multidisciplinary Approach to Understanding Cytomegalovirus Disease. Proceedings of the 4th international CMV workshop. Excerpta Medica. Amsterdam.311-320
(3) Liu Y-N, Klaus A, Kari B, Stinski M. F, Eckhardt J, and Gehrz R.C.(1991) The N-terminal 513 amino Acids of the Enveloppe Glycoprotein gB of Human Cytomegalovirus stimulates both B- and T-cell Immune responses in Humans. *J.Virol*. 65: 1654-1648
(4) Gilbert M. J, Riddell S. R, Li C-H, and Greenberg, P. (1993) Selective interference with class I Major histocompatibility complex presentation of the Major Immediate-Early Protein following Infection with Human Cytomegalovirus. *J. Virol*. 67: 3461-3469
(5) Davignon J-L, Clément D., Alriquet J., Michelson S., and Davrinche C (1994) Analysis of the proliferative T cell response specific for the Human Cytomegalovirus Protein IE1: Phenotype, Frequency and Variability. Scand. J. Immunol. 41:247-255
(6) Fattal, E., Couvreur, P., and Puisieux, F. (1993) Méthodes de préparation des liposomes In: Les Liposomes.eds.INSERM, Paris, France
(7) Ron, Y.,De Baestelier, P., Gordon, J., Feldman, M., and Segal, S. (1981) Defective induction of antigen-reactive proliferating T cells in B cell-deprived mice *Eur. J. Immunol*. 11 964
(8) Machy, P., and Leserman, L (1993) Internalisation des molécules de surface cellulaire: implications sur le ciblage des liposomes.ln: Les Liposomes.eds INSERM, Paris, France
(9). LaFemina, R. A., Pizzorno, M. C., Mosca, J. D., and Hayward, G. S. (1989): Expression of the acidic nuclear immediate early protein (IE1) of the human cytomegalovirusin stable cell lines and its preferential association with metaphase chromosome. *Virology.*172, 584-600
(10) Guan K, and Dixon J (1991) Eukariotic proteins expressed in *Escherichia Coli:* An improved thrombin cleavage and purification procedure of fusion proteins with glutathione S-transferase. *Analyt. Biorhem*. 192: 262-267
(11) Sambrook, J. , Fritsch, E., and Maniatis, T. (1989): Molecular cloning, a laboratory manual. Cold Spring Harbor Laboratory Mess.
(12) Sanger, F., Nicklen, S., and Coulson, A. R. (1977) DNA sequencing with chain terminating inhibitors. *Proc. Natl. Acad. Sci. USA* 74: 5463-5467
(13) Marston, F. A. O. (1986): The purification of eukariotic polypeptides synthetized in *Escherichia coli. Biochem. J*. 240:1-12.
(14) Smith, D. B. , and Johnson, K. S. (1988) Single-step purification of polypeptides expressed in *Escherichia coli* as fusions with glutathione-S-transferase. *Gene* 67:31-40.
(15) Laemmli, U. K. (1970): Cleavage of structural proteins during the assembly of the head of the bacteriophage T4. *Nature* 227:680-685
(16) Towbin, H. , Staehelin, T. , and Gordon, J. (1979): Electrophoretic transfer of proteins from polyacrylamide gels to nitrocellulose sheets : Procedure and some applications. *Proc. Natl. Acad. Sci. U. S. A.* 76:4350-4354.
(17) Stenberg, R., Depto, A. S., Fortney, J., and Nelson, J. (1989) Regulated expression of early and late RNAs and protein from the human cytomegalovirus immediate early gene region. *J. Virology* 63 2699-2708
(18) Davrinche C, Pasquier C, Cerutti M, Serradell, L Clement D, Devauchelle G, Michelson S, and Davignon J-L (1993) Expression of human Cytomegalovirus Immediate-early protein IE1 in insect cens: Splicing of RNA and recognition by CD4+ T-cell clones. *Biochem. Biophys. Res. Com.* 195 469-477

## Revendications

1. Procédé pour augmenter l'immunogénicité d'un antigène, **caractérisé en ce que** l'antigène est associé par des liaisons ioniques et/ou hydrophobes à un vecteur particulaire, ledit vecteur étant constitué soit (i) d'un noyau hydrophile non liquide, soit (ii) d'un noyau hydrophile non liquide comportant une couche externe de phospholipides, soit (iii) d'un noyau hydrophile non liquide comportant une couche externe d'acides gras.

2. Procédé selon la revendication 1, **caractérisé en ce que** le vecteur est constitué d'un noyau hydrophile non liquide.

3. Procédé selon la revendication 1, **caractérisé en ce que** le vecteur est constitué d'un noyau hydrophile non liquide comportant une couche externe de phospholipides naturels ou synthétiques.

4. Procédé selon la revendication 1, **caractérisé en ce que** le vecteur est constitué d'un noyau hydrophile non liquide comportant une couche externe d'acides gras.

5. Procédé selon la revendication 4, **caractérisé en ce que** la couche externe du vecteur est constituée d'acides gras naturels fixés au noyau par des liaisons covalentes.

6. Procédé selon l'une quelconque des revendications précédentes, **caractérisé en ce que** le noyau est constitué d'une matrice de polysaccharides ou d'oligosaccharides naturellement ou chimiquement réticulés.

7. Procédé selon l'une quelconque des revendications précédentes, **caractérisé en ce que** des ligands ioniques sont greffés sur le noyau.

8. Procédé selon la revendication 7, **caractérisé en ce que** le ligand ionique porte au moins sur une fonction choisie dans le groupe comprenant : phosphates, sulfates, acides carboxyliques, ammonium quaternaires, amines secondaires, amines primaires.

9. Procédé selon l'une des revendications 1 à 8, **caractérisé en ce que** l'antigène est associé au vecteur particulaire par des liaisons ioniques.

10. Procédé selon l'une des revendications 1 à 8, **caractérisé en ce que** l'antigène est associé au vecteur particulaire par des liaisons hydrophobes.

11. Procédé selon l'une quelconque des revendications 1 à 10, **caractérisé en ce que** l'antigène est associé au noyau du vecteur particulaire.

12. Procédé selon l'une quelconque des revendications 1 à 10, **caractérisé en ce que** l'antigène est associé à la couche externe du vecteur particulaire.

13. Produit susceptible d'être obtenu par le procédé selon l'une des revendications 1 à 12, comprenant une association d'un antigène lié par des liaisons ioniques et/ou hydrophobes à un vecteur particulaire constitué soit (i) d'un noyau hydrophile non liquide, soit (ii) d'un noyau hydrophile non liquide comportant une couche externe de phospholipides, soit (iii) d'un noyau hydrophile non liquide comportant une couche externe d'acides gras, **caractérisé en ce que** l'antigène est une protéine du CMV.

14. Produit la revendication 13, **caractérisé en ce que** l'antigène est le fragment e4 de la protéine IE1 du CMV.

15. Produit selon la revendication 14, **caractérisé en ce que** le fragment e4 est associé à la GST.

16. Produit selon l'une quelconque des revendications 13 à 15, **caractérisé en ce que** l'antigène est lié par des liaisons ioniques au vecteur particulaire.

17. Produit selon l'une quelconque des revendications 13 à 15, **caractérisé en ce que** l'antigène est lié par des liaisons hydrophobes au vecteur particulaire.

18. Produit selon Tune quelconque des revendications 13 à 17, **caractérisé en ce que** le vecteur particulaire est constitué d'un noyau hydrophile non liquide.

19. Produit selon l'une quelconque des revendications 13 à 17, **caractérisé en ce que** le vecteur particulaire est constitué d'un noyau hydrophile non liquide comportant une couche externe de phospholipides.

20. Produit selon l'une quelconque des revendications 13 à 17, **caractérisé en ce que** le vecteur particulaire est constitué d'un noyau hydrophile non liquide comportant une couche externe d'acides gras.

21. Utilisation d'un vecteur particulaire pour la préparation d'un médicament utile pour augmenter l'immunogénicité d'un antigène, ledit vecteur particulaire étant constitué soit (i) d'un noyau hydrophile non liquide, soit (ii) d'un noyau hydrophile non liquide comportant une couche externe de phospholipides, soit (iii) d'un noyau hydrophile non liquide comportant une couche externe d'acides gras, et l'antigène étant associé audit vecteur particulaire par des liaisons ioniques et/ou hydrophobes.

22. Utilisation selon la revendication 21, **caractérisé en ce que** l'antigène est lié par des liaisons ioniques au vecteur particulaire.

23. Utilisation selon la revendication 21, **caractérisé en ce que** l'antigène est lié par des liaisons hydrophobes au vecteur particulaire.

24. Utilisation selon l'une quelconque des revendications 21 à 23, **caractérisé en ce que** le vecteur particulaire est constitué d'un noyau hydrophile non liquide.

25. Utilisation selon l'une quelconque des revendications 21 à 23, **caractérisé en ce que** le vecteur particulaire est constitué d'un noyau hydrophile non liquide comportant une couche externe de phospholipides.

26. Utilisation selon l'une quelconque des revendications 21 à 23, **caractérisé en ce que** le vecteur particulaire est constitué d'un noyau hydrophile non liquide comportant une couche externe d'acides gras.

27. Utilisation selon l'une quelconque des revendications 21 à 26, **caractérisé en ce que** ledit médicament est un vaccin.

## Patentansprüche

1. Verfahren zur Erhöhung der Immunogenität eines Antigens, **dadurch gekennzeichnet, dass** das Antigen durch ionische und/oder hydrophobe Verbindungen an einen Partikelvektor angelagert ist, wobei der besagte Vektor entweder (i) aus einem hydrophilen, nicht flüssigen Kern oder (ii) einem hydrophilen, nicht flüssigen Kern mit einer äußeren Phospholipidschicht oder (iii) einem hydrophilen, nicht flüssigen Kern mit einer äußeren Fettsäureschicht besteht.

2. Verfahren nach Anspruch 1, **dadurch gekennzeichnet, dass** der Vektor aus einem hydrophilen, nicht flüssigen Kern besteht.

3. Verfahren nach Anspruch 1, **dadurch gekennzeichnet, dass** der Vektor aus einem hydrophilen, nicht flüssigen Kern mit einer äußeren Schicht natürlicher oder synthetischer Phospholipide besteht.

4. Verfahren nach Anspruch 1, **dadurch gekennzeichnet, dass** der Vektor aus einem hydrophilen, nicht flüssigen Kern mit einer äußeren Fettsäureschicht besteht.

5. Verfahren nach Anspruch 4, **dadurch gekennzeichnet, dass** die äußere Schicht des Vektors aus natürlichen Fettsäuren besteht, die am Kern durch kovalente Verbindungen fixiert sind.

6. Verfahren nach einem beliebigen der vorgenannten Ansprüche, **dadurch gekennzeichnet, dass** der Kern aus einer natürlich oder chemisch vernetzten Polysaccharid- bzw. Oligosaccharidmatrix besteht.

7. Verfahren nach einem beliebigen der vorgenannten Ansprüche, **dadurch gekennzeichnet, dass** ionische Liganden auf den Kern gepfropft sind.

8. Verfahren nach Anspruch 7, **dadurch gekennzeichnet, dass** der ionische Ligand mindestens auf eine, innerhalb der Gruppe gewählte Funktion einwirkt, zu der gehören: Phosphate, Sulfate, Carboxylsäuren, Quarternärammonium, sekundäre Amine, primäre Amine.

9. Verfahren nach einem der Ansprüche 1 bis 8, **dadurch gekennzeichnet, dass** das Antigen an den Partikelvektor durch ionische Verbindungen angelagert ist.

10. Verfahren nach einem der Ansprüche 1 bis 8, **dadurch gekennzeichnet, dass** das Antigen an den Partikelvektor durch hydrophobe Verbindungen angelagert ist.

11. Verfahren nach einem beliebigen der Ansprüche 1 bis 10, **dadurch gekennzeichnet, dass** das Antigen an den Kern des Partikelvektors angelagert ist.

12. Verfahren nach einem beliebigen der Ansprüche 1 bis 10, **dadurch gekennzeichnet, dass** das Antigen an die äußere Schicht des Partikelvektors angelagert ist.

13. Produkt, das durch das Verfahren nach einem der Ansprüche 1 bis 12 dargestellt werden kann, bestehend aus einer Anlagerung eines Antigens, das durch ionische und/oder hydrophobe Verbindungen mit einem Partikelvektor verbunden ist, der entweder (i) aus einem hydrophilen, nicht flüssigen Kern oder (ii) einem hydrophilen, nicht flüssigen Kern mit einer äußeren Phospholipidschicht oder (iii) einem hydrophilen, nicht flüssigen Kern mit einer äußeren Fettsäureschicht besteht, **dadurch gekennzeichnet, dass** das Antigen ein Protein des CMV ist.

14. Produkt nach Anspruch 13, **dadurch gekennzeichnet, dass** das Antigen das Fragment e4 des Proteins IEI des CMV ist.

15. Produkt nach Anspruch 14, **dadurch gekennzeichnet, dass** das Fragment e4 an die Glutathion-S-transferase angelagert ist.

16. Produkt nach einem beliebigen der Ansprüche 13 bis 15, **dadurch gekennzeichnet, dass** das Antigen durch ionische Verbindungen mit dem Partikelvektor verbunden ist.

17. Produkt nach einem beliebigen der Ansprüche 13 bis 15, **dadurch gekennzeichnet, dass** das Antigen durch hydrophobe Verbindungen mit dem Partikelvektor verbunden ist.

18. Produkt nach einem beliebigen der Ansprüche 13 bis 17, **dadurch gekennzeichnet, dass** der Partikelvektor aus einem hydrophilen, nicht flüssigen Kern besteht.

19. Produkt nach einem beliebigen der Ansprüche 13 bis 17, **dadurch gekennzeichnet, dass** der Partikelvektor aus einem hydrophilen, nicht flüssigen Kern mit einer äußeren Phospholipidschicht besteht.

20. Produkt nach einem beliebigen der Ansprüche 13 bis 17, **dadurch gekennzeichnet, dass** der Partikelvektor aus einem hydrophilen, nicht flüssigen Kern mit einer äußeren Fettsäureschicht besteht.

21. Einsatz eines Partikelvektors zur Herstellung eines Medikaments, das zur Erhöhung der Immunogenität eines Antigens dient, wobei der besagte Partikelvektor entweder (i) aus einem hydrophilen, nicht flüssigen Kern oder (ii) einem hydrophilen, nicht flüssigen Kern mit einer äußeren Phospholipidschicht oder (iii) einem hydrophilen, nicht flüssigen Kern mit einer äußeren Fettsäureschicht besteht, und das Antigen an den besagten Partikelvektor durch ionische und/oder hydrophobe Verbindungen angelagert ist.

22. Einsatz nach Anspruch 21, **dadurch gekennzeichnet, dass** das Antigen durch ionische Verbindungen mit dem Partikelvektor verbunden ist.

23. Einsatz nach Anspruch 21, **dadurch gekennzeichnet, dass** das Antigen durch hydropobe Verbindungen mit dem Partikelvektor verbunden ist.

24. Einsatz nach einem beliebigen der Ansprüche 21 bis 23, **dadurch gekennzeichnet, dass** der Partikelvektor aus einem hydrophilen, nicht flüssigen Kern besteht.

25. Einsatz nach einem beliebigen der Ansprüche 21 bis 23, **dadurch gekennzeichnet, dass** der Partikelvektor aus einem hydrophilen, nicht flüssigen Kern mit einer äußeren Phospholipidschicht besteht.

26. Einsatz nach einem beliebigen der Ansprüche 21 bis 23, **dadurch gekennzeichnet, dass** der Partikelvektor aus einem hydrophilen, nicht flüssigen Kern mit einer äußeren Fettsäureschicht besteht.

27. Einsatz nach einem beliebigen der Ansprüche 21 bis 26, **dadurch gekennzeichnet, dass** das besagte Medikament ein Impfstoff ist.

## Claims

1. Method for increasing the immunogenicity of an antigen, **characterized in that** the antigen is associated via ionic and/or hydrophobic bonds with a special vector, said vector being made up either of (i) a non-liquid hydrophilic nucleus, or (ii) a non-liquid hydrophilic nucleus comprising an outer layer of phospholipids, or (iii) a non-liquid hydrophilic nucleus comprising an outer layer of fatty acids.

2. Method according to claim 1, **characterized in that** the vector is made up of a non-liquid hydrophilic nucleus.

3. Method according to claim 1, **characterized in that** the vector is made of a non-liquid hydrophilic nucleus comprising an outer layer of natural or synthetic phospholipids.

4. Method according to claim 1, **characterized in that** the vector is made up of a non-liquid hydrophilic nucleus containing an outer layer of fatty acids.

5. Method according to claim 4, **characterized in that** the outer layer of the vector is made up of natural fatty acids attached to the nucleus by covalent bonds.

6. Method according to any of the preceding claims, **characterized in that** the nucleus is made up of a matrix of polysaccharides or oligosaccharides that are naturally or chemically cross-linked.

7. Method according to any of the preceding claims, **characterized in that** ionic ligands are grafted onto the nucleus.

8. Method according to claim 7, **characterized in that** the ionic ligand concerns at least one function chosen from the group comprising: phosphates, sulphates, carboxylic acids, quaternary ammonium, secondary amines, primary amines.

9. Method according to any of claims 1 to 8, **characterized in that** the antigen is associated with the special vector by ionic bonds.

10. Method according to any of claims 1 to 8, **characterized in that** the antigen is associated with the special vector by hydrophobic bonds.

11. Method according to any of claims 1 to 10, **characterized in that** the antigen is associated with the nucleus of the special vector.

12. Method according to any of claims 1 to 10, **characterized in that** the antigen is associated with the outer layer of the special vector.

13. Product able to be obtained using the method according to any of claims 1 to 12, containing an association of an antigen linked by ionic and/hydrophobic bonds to a special vector made up either of (i) a non-liquid hydrophilic nucleus, or (ii) a non-liquid hydrophilic nucleus comprising an outer layer of phospholipids, or (iii) a non-liquid hydrophilic nucleus comprising an outer layer of fatty acids, **characterized in that** the antigen is a CMV protein.

14. Product according to claim 13, **characterized in that** the antigen is the e4 fragment of the IE1 protein of CMV.

15. Product according to claim 14, **characterized in that** the e4 fragment is associated with GST.

16. Product according to any of claims 13 to 15, **characterized in that** the antigen is linked by ionic bonds to the special vector.

17. Product according to any of claims 13 to 15, **characterized in that** the antigen is linked by hydrophobic bonds to the special vector.

18. Product according to any of claims 13 to 17, **characterized in that** the special vector is made up of a non-liquid hydrophilic nucleus.

19. Product according to any of claims 13 to 17, **characterized in that** the special vector is made up of a non-liquid hydrophilic nucleus comprising an outer layer of phospholipids.

20. Product according to any of claims 13 to 17, **characterized in that** the special vector is made up of a non-liquid hydrophilic nucleus comprising an outer layer of fatty acids.

21. Use of a special vector to prepare a medicinal product which can be used to increase the immunogenicity of an antigen, said special vector being made up either of (i) a non-liquid hydrophilic nucleus, or (ii) a non-liquid hydrophilic nucleus comprising an outer layer of phospholipids, or (iii) a non-liquid hydrophilic nucleus comprising an outer layer of fatty acids, and the antigen being associated with said special vector by ionic and/or hydrophobic bonds.

22. Use according to claim 21, **characterized in that** the antigen is linked by ionic bonds to the special vector.

23. Use according to claim 21, **characterized in that** the antigen is linked by hydrophobic bonds to the special vector.

24. Use according to any of claims 21 to 23, **characterized in that** the special vector is made up of a non-liquid hydrophilic nucleus.

25. Use according to any of claims 21 to 23, **characterized in that** the special vector is made up of a non-liquid hydrophilic nucleus comprising an outer layer of phospholipids.

26. Use according to any of claims 21 to 23, **characterized in that** the special vector is made up of a non-liquid hydrophilic nucleus comprising an outer layer of fatty acids.

27. Use according to any of claims 21 to 26, **characterized in that** said medicinal product is a vaccine.
